# EUROPEAN PATENT SPECIFICATION

(11) **EP 3 071 170 B1**
(45) Date of publication and mention of the grant of the patent: **28.02.2018**
(21) Application number: 13812239.5
(22) Date of filing: 22.11.2013
(51) Int. Cl.: A61Q 11/00, A61K 8/46, A61K 8/25, A61K 8/26, A61K 8/44

(54) **TOOTH WHITENING ORAL CARE PRODUCT**
ZAHNAUFHELLENDES MUNDPFLEGEPRODUKT
PRODUIT D'HYGIÈNE ORALE POUR LE BLANCHIMENT DES DENTS

(43) Date of publication of application: 28.09.2016
(73) Proprietor: Colgate-Palmolive Company, New York, NY 10022 (US)
(72) Inventor: FEI, Lin, Kendall Park, New Jersey 08824 (US); CHOPRA, Suman K., Monroe, New Jersey 08831 (US)
(74) Representative: Wibbelmann, Jobst
(86) International application number: PCT/US2013/071387
(87) International publication number: WO 2015/076817

(56) References cited:
- WO-A1-2013/095435
- JP-A- 2006 151 877
- US-A- 6 106 812
- DATABASE GNPD [Online] MINTEL; January 2008 (2008-01), "Advanced Whitening Toothpaste", XP002726620, Database accession no. 845360
- J. CENENS ET AL: "Visible Spectroscopy of Methylene Blue on Hectorite, Laponite B, and Barasym in Aqueous Suspension", CLAYS AND CLAY MINERALS, vol. 36, no. 3, 1 January 1988 (1988-01-01), pages 214-224, XP055127094, ISSN: 0009-8604, DOI: 10.1346/CCMN.1988.0360302
- ROULIA ET AL: "Interactions between C.I. Basic Blue 41 and aluminosilicate sorbents", JOURNAL OF COLLOID AND INTERFACE SCIENCE, ACADEMIC PRESS, NEW YORK, NY, US, vol. 291, no. 1, 1 November 2005 (2005-11-01), pages 37-44, XP005086635, ISSN: 0021-9797, DOI: 10.1016/J.JCIS.2005.04.085

## Description

### BACKGROUND

Many people desire whiter teeth, and there are tooth whitening products for that purpose. For example, using pigments with a polymer in toothpaste to make teeth look whiter is a technology used in markets outside of the US. In the US, however, only dye is allowed in oral care products. A significant problem of using water soluble dye is that the dye will be quickly dissolved by saliva, and cannot provide durable effect as pigment, In the US, peroxide-based tooth whitening products are common, but approved levels of peroxide vary from country to country, and such products may be difficult to formulate due to the tendency of hydrogen peroxide to oxidize other ingredients in the formulation and to decompose into water and oxygen.

WO 2012/016908 discloses certain oral care compositions containing lake dyes formed by dissolving an aluminum salt in water and adding an alkali to the solution to precipitate the hydrated alumina, followed by addition of a taking agent, typically aluminum chloride. The dye is fixed to an inorganic substrate such as calcium carbonate, barium sulphate, kaolin, talc and certain metal oxides. However, these substrates are not optimized for adsorption of the dye or for retention on the teeth.

Many people suffer from tooth sensitivity due to the exposure of dentin, the part of the tooth which covers the nerve, either through loss of the enamel layer or recession of the gums. The dentin contains a large numbers of microtubules that run from the outside of the tooth to the nerve in the center. When the dentin is exposed, the microtubules can transmit stimuli, e.g., from changes in temperature or from certain foods (acidic or sweet) to the nerve, causing the tooth or teeth to be painful. The pain usually subsides after a short period of time.

Tooth whitening is a particular problem for a person having sensitize teeth, as some whitening products utilize harsh chemicals that may cause pain or exacerbate tooth sensitivity. Despite the many products on the market and the many efforts to address these problems over the years, there still remains a need for safe and effective alternative oral care products to address the problems of dental hypersensitivity and tooth whitening.

### BRIEF SUMMARY

The present invention concerns a tooth whitening oral care composition comprising:
(i) a whitening complex, wherein the complex comprises
   (a) a magnesium alkali metal silicate clay, wherein the clay is a hectorite clay; and
   (b) triarylmethane dye as an orally acceptable blue dye,
(ii) an orally acceptable carrier; and
(iii) L-arginine in free or orally acceptable salt form;
wherein the complex is prepared by:
dispersing the clay into an aqueous solution, combining the aqueous solution of clay with an aqueous solution of the dye, adding salt, optionally sodium chloride, under agitation, and isolating the complex from the liquid phase.
Further embodiments of the invention are set forth in greater detail in the attached claims.

Further areas of applicability of the present invention will become apparent from the detailed description provided hereinafter. It should be understood that the detailed description and specific examples, while indicating the preferred embodiment of the invention, are intended for purposes of illustration only and are not intended to limit the scope of the invention.

### DETAILED DESCRIPTION

The following description of the preferred embodiment(s) is merely exemplary in nature and is in no way intended to limit the invention, its application, or uses.

### Orally acceptable blue dyes

Orally acceptable blue dyes are blue dyes which are safe for use in oral care applications, and include blue dyes from natural sources as well as synthetic dyes approved for use in foods or oral care products, e.g. FD&C Blue No. 1 and FD&C Blue No. 2. Dyes for use in the present invention to prepare the water insoluble whitening complex may be water soluble. The term "water-soluble" in this particular context generally means that the dye has an aqueous solubility of at least 10 g/L at 25°C., most preferably at least 100 g/L at 25°C. (where the solubility is determined in un-buffered distillled water).

In particular embodiments, aqueous solutions of dyes useful herein have a maximum absorbance value in the visible spectrum (λₘₐₓ) at a wavelength ranging from 550 to 650, more preferably from 600 to 650 nm. Dyes useful herein may have a blue to blue-green color with a hue angle in the CIELAB system ranging from 180 to 270 degrees, more particularly 180 to 200 degrees. Dyes useful herein include anionic triphenylmethane dyes, and especially diaminotriphenylmethane dyes containing from two to four sulphonate groups, such as those corresponding to general formula (I): 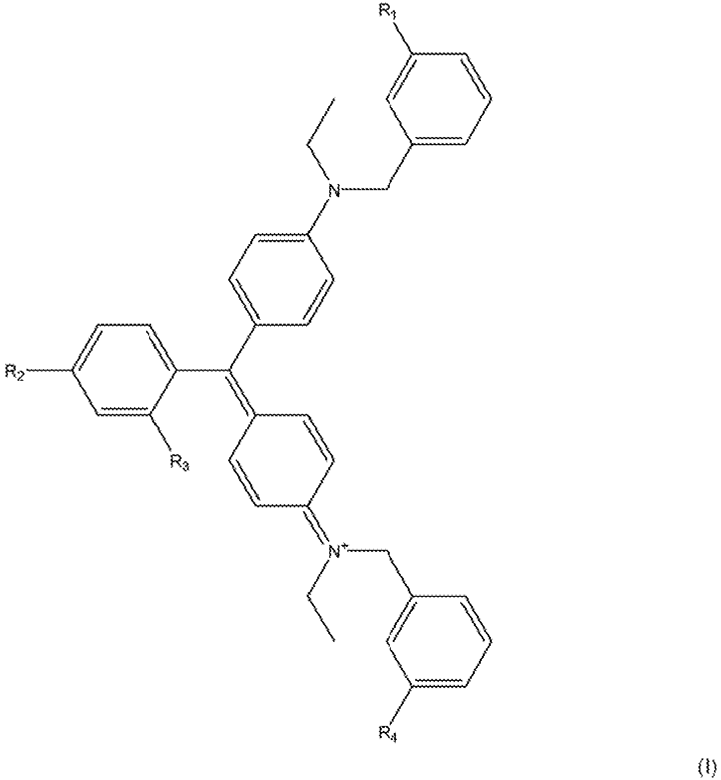 in which R₁, R₂, R₃ and R₄ are monovalent moieties which are each independently selected from hydrogen (-H), hydroxyl (-OH), halo (e.g. -Cl) and sulphonate (-SO₃⁻) groups, with the proviso that at least two of R₁ to R₄ are sulphonate groups.

An example of a dye useful herein is FD&C Blue #1, also known as Brilliant Blue FCF (Blue 1) as well as other commercial names, which corresponds to general formula (I), wherein R₂ is -H and R₁, R₃, and R₄ are sulphonate groups. FD&C Blue #1 is a colorant for foods and other substances to induce a color change. It is denoted by E number E133 and has a color index of 42090. It has the appearance of a reddish-blue powder. It is soluble in water, and the solution has a maximum absorption at about 628 nanometers. It is a synthetic dye produced using aromatic hydrocarbons from petroleum. It is usually a disodium salt. The diammonium salt has CAS number [2650-18-2]. Calcium and potassium salts are also known.

Additional dyes may be used in conjunction with the blue dye, in order to adjust the precise color absorption as desired.

*Magnesium Alkali Metal Silicate Clay:* Alkali metal silicate clays for use in the invention are magnesium alkali metal silicate complex clays. One such is hectorite, which is a smectite clay, belonging to a family of layered minerals that are comprised of very small individual platelets with a metal oxide center sandwiched between two silicone dioxide outer layers. Hectorite is a trioctahedral, magnesium based clay, e.g., typically having an approximate Chemical composition of Na_{0.3}(Mg,Li)₃Si₄O₁₀(OH)₂.

It forms small, elongated disc-shaped particles, about 1 nm in thickness, and about 20 nm in diameter. These particles thus provide a very high surface area. Synthetic hectorite is a synthetic colloidal magnesium alkali metal silicate complex clay commercially available under the trade designation, Laponite®, e.g., from Southern Clay Products, Inc. Laponites are synthetic hectorite clays composed of magnesium, lithium, silica, oxygen, hydrogen, and sodium. Like natural hectorites, Laponites are composed in the dry state of platelets, about 1 nm thick, arranged in stacks. Each platelet has a double layer of tetrahedral silica bonded to oxygen atoms. Between the two silica layers there is a sheet of cations composed of magnesium and lithium in approximately a 5.3 to 0.7 ratio. These cations coordinate the inner row of silica bound oxygens and OH groups. In the presence of water, the cations create an osmotic gradient, causing the platelets to swell, forming a gel. Laponite® XLG is a product which has been processed to remove trace heavy metals and thus is particularly suitable for application in the instant invention. Laponite® XLG material contains 59.5% of silicon dioxide, 27.5% magnesium oxide, 0.8% dilithium oxide and 2.8% disodium oxide. It forms highly thixotropic gels when mixed in water.

These clays, having relatively high levels of magnesium and being free or nearly free of aluminum, are distinct from clays having high levels of aluminum, such as kaolinite (Al₂Si₂O₅(OH)₄), a white clay often used for making ceramics, or bentonite, which is primarily composted of montmorillonite having the formula (Na,Ca)_{0.33}(Al,Mg)₂(Si₄O₁₀)(OH)₂·*n*H₂O.

*Water Insoluble Whitening Complex:* The water insoluble whitening complex of the invention comprises a complex of the water soluble triarylmethane dye and the magnesium alkali metal silicate clay. Such complexes can be prepared by dispersing the clay into an aqueous solution which is combined with an aqueous solution of the dye. A suitable salt such as sodium chloride is added under agitation. Under these conditions the dye complexes with the clay to form the water insoluble whitening complex. The water insoluble whitening complex can be isolated from the liquid phase by any suitable means known in the art, for example, centrifugation, filtration, and the like. Typically, more than 80%, more particularly at least 90% of the triarylmethane dye is absorbed onto the alkali silicate clay when forming the insoluble whitening complexes when the weight ratio of dye:clay is 0.05:1. Also, once the insoluble complex is formed, less than 50% of the absorbed triarylmethane dye is desorbed when dispersed in deionized water, more particularly less than 20%, more particularly less than 10%.

The blue dye typically comprises about 0.1 to 20 %, more particularly about 1 to 10%, and more particularly about 2 to 8 % of the water insoluble whitening complex. Conversely, the magnesium alkali metal silicate clay typically comprises about 80 to 99.9%, more particularly about 90 to 99%, and more particularly about 92 to 98% of the water insoluble whitening complex.

The water insoluble whitening complex typically comprises about 0.01 to 5%, more typically about 0.015 to 5%, more particularly about 0.015 to 3% of the composition of the invention.

*Orally Acceptable Polymers:* The oral care compositions of the invention may also include one or more anionic or nonionic polymers which may enhance the effect of the water insoluble whitening complex, and also may include additional polymers to adjust the viscosity of the formulation or enhance the solubility of other ingredients. Such polymers may function as deposition aids, that is, polymers which aid deposition of the water insoluble whitening complex from the composition onto the surface of teeth during use of the composition. Polymer deposition aids work by having affinity for both the dye water insoluble whitening complex and the surface of the teeth. The term "orally-acceptable" refers to a polymer which can be used to applied the to the oral cavity in a safe manner during formal use.

Suitable polymers include polyethylene glycols (PEGs), polypropylene glycols (PPGs), PEG/PPG copolymers, polysaccharides (e.g., cellulose derivatives, for example carboxymethyl cellulose, or polysaccharide gums, for example xanthan gum or carrageenan gum). Acidic polymers, for example polyacrylate gels, may be provided in the form of their free acids or partially or fully neutralized water soluble alkali metal (e.g., potassium and sodium) or ammonium salts. Anionic polymers useful herein may enhance the effect of the water insoluble whitening complex, for example in an amount of from about 0.001 to about 5%, more particularly about 0.01 to 5%, more particularly about 0.05 to 4%, more particularly about 0.05 to 3% of the composition. Such agents are known generally for use in dentifrice, although not for this particular application, useful in the present invention are disclosed in U.S. Pat. Nos. 5,188,821 and 5,192,531; and include synthetic anionic polymeric polycarboxylates, such as 1:4 to 4:1 copolymers of maleic anhydride or acid with another polymerizable ethylenically unsaturated monomer, preferably methyl vinyl ether/maleic anhydride having a molecular weight (M.W.) of about 30,000 to about 1,000,000, most preferably about 300,000 to about 800,000. These copolymers are available for example as Gantrez. e.g., AN 139 (M.W. 500,000), AN 119 (M.W. 250,000) and preferably S-97 Pharmaceutical Grade (M.W. 700,000) available from Ashland Specialty Chemicals, Bound Brook, N.J. 08805. Other operative polymers include those such as the 1:1 copolymers of maleic anhydride with ethyl acrylate, hydroxyethyl methacrylate, N-vinyl-2-pyrollidone, or ethylene, the latter being available for example as Monsanto EMA No. 1103, M.W. 10,000 and EMA Grade 61, and 1:1 copolymers of acrylic acid with methyl or hydroxyethyl methacrylate, methyl or ethyl acrylate, isobutyl vinyl ether or N-vinyl-2-pyrrolidone. Suitable generally, are polymerized olefinically or ethylenically unsaturated carboxylic acids containing an activated carbon-to-carbon olefinic double bond and at least one carboxyl group, that is, an acid containing an olefinic double bond which readily functions in polymerization because of its presence in the monomer molecule either in the alpha-beta position with respect to a carboxyl group or as part of a terminal methylene grouping. Illustrative of such acids are acrylic, methacrylic, ethacrylic, alpha-chloroacrylic, crotonic, beta-acryloxy propionic, sorbic, alpha-chlorsorbic, cinnamic, beta-styrylacrylic, muconic, itaconic, citraconic, mesaconic, glutaconic, aconitic, alpha-phenylacrylic, 2-benzyl acrylic, 2-cyclohexylacrylic, angelic, umbellic, fumaric, maleic acids and anhydrides. Other different olefinic monomers copolymerizable with such carboxylic monomers include vinylacetate, vinyl chloride, dimethyl maleate and the like. Copolymers contain sufficient carboxylic salt groups for water-solubility. A further class of polymeric agents includes a composition containing homopolymers of substituted acrylamides and/or homopolymers of unsaturated sulfonic acids and salts thereof, in particular where polymers are based on unsaturated sulfonic acids selected from acrylamidoalykane sulfonic acids such as 2-acrylamide 2 methylpropane sulfonic acid having a molecular weight of about 1,000 to about 2,000,000, described in U.S. Pat. No. 4,842,847, Jun. 27, 1989 to Zahid, incorporated herein by reference. Another useful class of polymeric agents includes polyamino acids containing proportions of anionic surface-active amino acids such as aspartic acid, glutamic acid and phosphoserine, (in addition to the basic amino acid polymers), e.g. as disclosed in U.S. Pat. No. 4,866,161 Sikes et al., incorporated herein by reference.

In preparing oral care compositions, it is sometimes necessary to add some thickening material to provide a desirable consistency or to stabilize or enhance the performance of the formulation. In certain embodiments, the thickening agents are carboxyvinyl polymers, carrageenan, hydroxyethyl cellulose and water soluble salts of cellulose ethers such as sodium carboxymethyl cellulose and sodium carboxymethyl hydroxyethyl cellulose. Natural gums such as karaya, gum arabic, and gum tragacanth can also be incorporated. Colloidal magnesium aluminum silicate or finely divided silica can be used as component of the thickening composition to further improve the composition's texture. In certain embodiments, thickening agents in an amount of about 0.05% to 5%, more particularly about 0.5 to 5% by weight of the total composition are used.

Orally acceptable carrier polymers for use in the invention are typically water soluble. The term "water-soluble" in this particular context generally means that the polymer has an aqueous solubility of at least 10 g/L at 25°C., and more preferably at least 30 g/L at 25°C. (where the solubility is determined in un-buffered distilled water). It is preferable that the polymer remains water soluble after drying, so that it can be re-dissolved. This prevents undesirable build up of the polymer on the teeth after repeated usage of the composition.

Certain orally acceptable carrier polymers are able to aid the deposition of the insoluble whitening complex onto the teeth such that tooth surface whiteness is enhanced by at least 20% and more preferably by at least 100%, in comparison to the value obtained for teeth treated in an equivalent manner with a control formulation using the same amount of triarylmethane dye in the absence of the orally acceptable carrier polymers. A method for determining tooth whiteness is described in WO 2012/016908.

Suitable orally acceptable carrier polymers for use in the invention will generally dissolve or disperse in water at a temperature of 25°C.

The amount of orally acceptable carrier polymer in compositions of the invention, whether enhancers, deposition aids, thickeners of a combination thereof, suitably ranges from about 0.001 to 5%, more particularly about 0.005 to 5%, more particularly about 1 to 5%, and more particularly about 1 to 3%.

*Liquid Continuous Phase:* Depending on the product form, the composition of the invention may comprise a continuous phase comprising water or polyhydric alcohol or a mixture thereof. For example, a dentifrice composition according to the invention will usually contain a liquid continuous phase in an amount of from 40 to 99% by weight based on the total weight of the dentifrice. Such a liquid continuous phase will typically comprise a mixture of water and polyhydric alcohol in various relative amounts, with the amount of water generally ranging from 10 to 45% by weight (based on the total weight of the dentifrice) and the amount of polyhydric alcohol generally ranging from 30 to 70% by weight (based on the total weight of the dentifrice). Typical polyhydric alcohols include humectants such as glycerol, sorbitol, polyethylene glycol, polypropylene glycol, propylene glycol, xylitol (and other edible polyhydric alcohols), hydrogenated partially hydrolyzed polysaccharides and mixtures thereof.

Water, employed in the preparation of commercial oral compositions should be deionized and free of organic impurities. Water commonly makes up the balance of the compositions and includes about 0.1% to about 90%, about 10% to about 80% or about 20% to about 70%, or about 30% to about 60% by weight of the oral compositions. This amount of water includes the free water which is added plus that amount which is introduced with other materials such as with sorbitol or any components of the invention.

*Product Form :* Examples of suitable product forms for compositions of the invention include dentifrices, mouthwashes, chewing gums and lozenges.

A particular product form useful to implement the present invention is a dentifrice. The term "dentifrice" generally denotes formulations that are used to clean the surfaces of the oral cavity. The dentifrice is an oral composition that is not intentionally swallowed for purposes of systemic administration of therapeutic agents, but is applied to the oral cavity, used to treat the oral cavity and then expectorated. Typically the dentifrice is used in conjunction with a cleaning implement such as a toothbrush, usually by applying it to the bristles of the toothbrush and then brushing the accessible surfaces of the oral cavity. Preferably the dentifrice is in the form of a paste or a gel (or a combination thereof).

Another type of product form in the context of the present invention is a mouthwash. The term "mouthwash" generally denotes liquid formulations which are used to rinse the surfaces of the oral cavity and provide the user with a sensation of oral cleanliness and refreshment. The mouthwash is an oral composition that is not intentionally swallowed for purposes of systemic administration of therapeutic agents, but is applied to the oral cavity, used to treat the oral cavity and then expectorated. A mouthwash composition according to the invention will usually contain an aqueous continuous phase. The amount of water generally ranges from 70 to 99% by weight based on the total weight ot the mouthwash.

*Active Agents*: The effective concentration of the active ingredients used herein will depend on the particular agent and the delivery system used. It is understood that a toothpaste for example will typically be dilated with water upon use, while a mouth rinse typically will not be. Thus, an effective concentration of active in a toothpaste will ordinarily be 5-15x higher than required for a mouth rinse. The concentration will also depend on the exact salt or polymer selected. For example, where the active agent is provided in salt form, the counterion will affect the weight of the salt, so that if the counterion is heavier, more salt by weight will be required to provide the same concentration of active ion in the final product. Arginine, where present, may be present at levels from, e.g., about 0.1 to about 20 wt %(expressed as weight of free base), e.g., about 0.1 to about 3 wt % for a mouthrinse, about 1 to about 10 wt % for a consumer toothpaste or about 7 to about 20 wt % for a professional or prescription treatment product. Fluoride where present may be present at levels of, e.g., about 25 to about 25,000 ppm, for example about 25 to about 250 ppm for a mouthrinse, about 750 to about 2,000 ppm for a consumer toothpaste, or about 2,000 to about 25,000 ppm for a professional or prescription treatment product. Levels of antibacterial agents in addition to the gallium salt and basic amino acid polymer will vary similarly, with levels used in toothpaste being e.g., about 5 to about 15 times greater than used in mouthrinse. For example, a triclosan mouthrinse may contain, e.g., about 0.03 wt % triclosan while a triclosan toothpaste may contain about 0.3 wt % triclosan.

*Fluoride Ion Source*: The oral care compositions may further include one or more fluoride ion sources, e.g., soluble fluoride salts. A wide variety of fluoride ion-yielding materials can be employed as sources of soluble fluoride in the present compositions. Examples of suitable fluoride ion-yielding materials are found in U.S. Pat. No. 3,535,421, to Briner et al.; U.S. Pat. No. 4,885,155, to Parran, Jr. et al. and U.S. Pat. No. 3,678,154, to Widder et al., incorporated herein by reference. Representative fluoride ion sources include, but are not limited to, stannous fluoride, sodium fluoride, potassium fluoride, sodium monofluorophosphate, sodium fluorosilicate, ammonium fluorosilicate, amine fluoride, ammonium fluoride, and combinations thereof. In certain embodiments the fluoride ion source includes stannous fluoride, sodium fluoride, sodium monofluorophosphate as well as mixtures thereof. In certain embodiments, the oral care composition of the invention may also contain a source of fluoride ions or fluorine-providing ingredient in amounts sufficient to supply about 25 ppm to about 25,000 ppm of fluoride ions, generally at least about 500 ppm, e.g., about 500 to about 2000 ppm, e.g., about 1000 to about 1600 ppm, e.g., about 1450 ppm. The appropriate level of fluoride will depend on the particular application. A toothpaste for general consumer use would typically have about 1000 to about 1500 ppm, with pediatric toothpaste having somewhat less. A dentifrice or coating for professional application could have as much as about 5,000 or even about 25,000 ppm fluoride. Fluoride ion sources may be added to the compositions of the invention at a level of about 0.01 wt. % to about 10 wt. % in one embodiment or about 0.03 wt. % to about 5 wt. %, and in another embodiment about 0.1 wt. % to about 1 wt. % by weight of the composition in another embodiment. Weights of fluoride salts to provide the appropriate level of fluoride ion will obviously vary based on the weight of the counter ion in the salt.

*Abrasives*: The compositions of the invention, e.g. Composition 1 et seq. may comprise a calcium phosphate abrasive, e.g., tricalcium phosphate (Ca₃(PO₄)₂), hydroxyapatite (Ca₁₀(PO₄)₆(OH)₂), or dicalcium phosphate dihydrate (CaHPO₄ • 2H₂O, also sometimes referred to herein as DiCal) or calcium pyrophosphate. The compositions may include one or more additional abrasives, for example silica abrasives such as precipitated silicas having a mean particle size of up to about 20 microns, such as Zeodent 115®, marketed by J. M. Huber. Other useful abrasives also include sodium metaphosphate, potassium metaphosphate, aluminum silicate, calcined alumina, bentonite or other siliceous materials, or combinations thereof, The silica abrasive polishing materials useful herein, as well as the other abrasives, generally have an average particle size ranging between about 0.1 and about 30 microns, about between 5 and about 15 microns. The silica abrasives can be from precipitated silica or silica gels, such as the silica xerogels described in U.S. Pat. No. 3,538,230, to Pader et al. and U.S. Pat. No. 3,862,307, to Digiulio, both incorporated herein by reference. Particular silica xerogels are marketed under the trade name Syloid® by the W. R. Grace & Co., Davison Chemical Division. The precipitated silica materials include those marketed by the J. M. Huber Corp. under the trade name Zeodent®, including the silica carrying the designation Zeodent 115 and 119. These silica abrasives are described in U.S. Pat. No. 4,340,583, to Wason, incorporated herein by reference. In certain embodiments, abrasive materials useful in the practice of the oral care compositions in accordance with the invention include silica gels and precipitated amorphous silica having an oil absorption value of less than about 100 cc/100 g silica and in the range of about 45 cc/100 g to about 70 cc/100 g silica. Oil absorption values are measured using the ASTA Rub-Out Method D281. In certain embodiments, the silicas are colloidal particles having an average particle size of about 3 microns to about 12 microns, and about 5 to about 10 microns. Low oil absorption silica abrasives particularly useful in the practice of the invention are marketed under the trade designation Sylodent XWA® by Davison Chemical Division of W.R. Grace & Co., Baltimore, Md. 21203. Sylodent 650 XWA®, a silica hydrogel composed of particles of colloidal silica having a water content of 29% by weight averaging about 7 to about 10 microns in diameter, and an oil absorption of less than about 70 cc/100 g of silica is an example of a low oil absorption silica abrasive useful in the practice of the present invention. The abrasive is present in the oral care composition of the present invention at a concentration of about 10 to about 60% by weight, in other embodiment about 20 to about 45% by weight, and in another embodiment about 30 to about 50% by weight.

*Foaming Agents*: The oral care compositions of the invention also may include an agent to increase the amount of foam that is produced when the oral cavity is brushed. Illustrative examples of agents that increase the amount of foam include, but are not limited to polyoxyethylene and certain polymers including, but not limited to, alginate polymers. The polyoxyethylene may increase the amount of foam and the thickness of the foam generated by the oral care carrier component of the present invention. Polyoxyethylene is also commonly known as polyethylene glycol ("PEG") or polyethylene oxide. The polyoxyethylenes suitable for this invention will have a molecular weight of about 200,000 to about 7,000,000. In one embodiment the molecular weight will be about 600,000 to about 2,000,000 and in another embodiment about 800,000 to about 1,000,000. Polyox® is the trade name for the high molecular weight polyoxyethylene produced by Union Carbide. The polyoxyethylene may be present in an amount of about 1% to about 90%, in one embodiment about 5% to about 50% and in another embodiment about 10% to about 20% by weight of the oral care carrier component of the oral care compositions of the present invention. The dosage of foaming agent in the oral care composition (i.e., a single dose) is about 0.01 to about 0.9 % by weight, about 0.05 to about 0.5% by weight, and in another embodiment about 0.1 to about 0.2 % by weight.

*Surfactants*: The compositions useful in the invention may contain anionic surfactants, for example:
i. water-soluble salts of higher fatty acid monoglyceride monosulfates, such as the sodium salt of the monosulfated monoglyceride of hydrogenated coconut oil fatty acids such as sodium N-methyl N-cocoyl taurate, sodium cocomonoglyceride sulfate,
ii. higher alkyl sulfates, such as sodium lauryl sulfate,
iii. higher alkyl-ether sulfates, e.g., of formula CH₃(CH₂)ₘCH₂(OCH₂CH₂)ₙOSO₃X, wherein m is 6-16, e.g., 10, n is 1-6, e.g., 2, 3 or 4, and X is Na or K, for example sodium laureth-2 sulfate (CH₃(CH₂)₁₀CH₂(OCH₂CH₂)₂OSO₃Na).
iv. higher alkyl aryl sulfonates such as sodium dodecyl benzene sulfonate (sodium lauryl benzene sulfonate)
v. higher alkyl sulfoacetates, such as sodium lauryl sulfoacetate (dodecyl sodium sulfoacetate), higher fatty acid esters of 1,2 dihydroxy propane sulfonate, sulfocolaurate (N-2-ethyl laurate potassium sulfoacetamide) and sodium lauryl sarcosinate.

By "higher alkyl" is meant, e.g., C₆₋₃₀ alkyl. In particular embodiments, the anionic surfactant is selected from sodium lauryl sulfate and sodium ether lauryl sulfate. The anionic surfactant may be present in an amount which is effective, e.g., > 0.01% by weight of the formulation, but not at a concentration which would be irritating to the oral tissue, e.g., <10%, and optimal concentrations depend on the particular formulation and the particular surfactant. For example, concentrations used for a mouthwash are typically on the order of one tenth that used for a toothpaste. In one embodiment, the anionic surfactant is present in a toothpaste at from about 0.3% to about 4.5% by weight, e.g., about 1.5%. The compositions of the invention may optionally contain mixtures of surfactants, e.g., comprising anionic surfactants and other surfactants that may be anionic, cationic, zwitterionic or nonionic. Generally, surfactants are those which are reasonably stable throughout a wide pH range. Surfactants are described more fully, for example, in U.S. Pat. No. 3,959,458, to Agricola et al.; U.S. Pat. No. 3,937,807, to Haefele; and U.S. Pat. No. 4,051,234, to Gieske et al, which are incorporated herein by reference. In certain embodiments, the anionic surfactants useful herein include the water-soluble salts of alkyl sulfates having about 10 to about 18 carbon atoms in the alkyl radical and the water-soluble salts of sulfonated monoglycerides of fatty acids having about 10 to about 18 carbon atoms. Sodium lauryl sulfate, sodium lauryl sarcosinate and sodium coconut monoglyceride sulfonates are examples of anionic surfactants of this type. In a particular embodiment, the composition of the invention, e.g., Composition 1, et seq., comprises sodium lauryl sulfate.

The surfactant or mixtures of compatible surfactants can be present in the compositions of the present invention in about 0.1% to about 5.0%, in another embodiment about 0.3% to about 3.0% and in another embodiment about 0.5% to about 2.0% by weight of the total composition.

*Flavoring Agents*: The oral care compositions of the invention may also include a flavoring agent. Flavoring agents which are used in the practice of the present invention include, but are not limited to, essential oils as well as various flavoring aldehydes, esters, alcohols, and similar materials. Examples of the essential oils include oils of spearmint, peppermint, wintergreen, sassafras, clove, sage, eucalyptus, marjoram, cinnamon, lemon, lime, grapefruit, and orange. Also useful are such chemicals as menthol, carvone, and anethole. Certain embodiments employ the oils of peppermint and spearmint. The flavoring agent may be incorporated in the oral composition at a concentration of about 0.1 to about 5% by weight and about 0.5 to about 1.5% by weight. The dosage of flavoring agent in the individual oral care composition dosage (i.e., a single dose) is about 0.001 to 0.05% by weight and in another embodiment about 0.005 to about 0.015 % by weight.

*Humectants*: Within certain embodiments of the oral compositions, it is also desirable to incorporate a humectant to prevent the composition from hardening upon exposure to air. Certain humectants can also impart desirable sweetness or flavor to dentifrice compositions. The humectant, on a pure humectant basis, generally includes about 15% to about 70% in one embodiment or about 30% to about 65% in another embodiment by weight of the dentifrice composition. Suitable humectants include edible polyhydric alcohols such as glycerine, sorbitol, xylitol, propylene glycol as well as other polyols and mixtures of these humectants. Mixtures of glycerine and sorbitol may be used in certain embodiments as the humectant component of the toothpaste compositions herein.

*Other optional ingredients*: In addition to the above-described components, the embodiments of this invention can contain a variety of optional dentifrice ingredients some of which are described below. Optional ingredients include, for example, but are not limited to, adhesives, sudsing agents, flavoring agents, sweetening agents, antiplaque agents, abrasives, and additional coloring agents. These and other optional components are further described in U.S. Pat. No. 5,004,597, to Majeti; U.S. Pat. No. 3,959,458 to Agricola et al. and U.S. Pat. No. 3,937,807, to Haefele, all being incorporated herein by reference.

As used throughout, ranges are used as shorthand for describing each and every value that is within the range. Any value within the range can be selected as the terminus of the range. In addition, all references cited herein are hereby incorporated by referenced in their entireties. In the event of a conflict in a definition in the present disclosure and that of a cited reference, the present disclosure controls.

Unless otherwise specified, all percentages and amounts expressed herein and elsewhere in the specification should be understood to refer to percentages by weight. The amounts given are based on the active weight of the material. For convenience, components of the composition of invention are expressed in the singular; however it is to be understood that mixtures of components are encompassed by use of the singular expression, for example, "an orally acceptable carrier polymer" may include mixtures of two or more polymers described herein.

The invention thus provides, in one embodiment, an oral care composition (Composition 1), e.g. a dentifrice composition, comprising (i) a water insoluble whitening complex comprising
(a) an orally acceptable blue dye, e.g, a triarylmethane dye, and (b) a magnesium alkali metal silicate clay, and (ii) an orally acceptable carrier; for example,
   1.1. Composition 1 wherein the magnesium alkali metal silicate clay is a hectorite clay.
   1.2. Composition 1.1 wherein the hectorite clay is a synthetic clay comprising 58-61% silicon dioxide, 26-29% magnesium oxide, 0.7-0.9% dilithium oxide and 2.6-3% disodium oxide, e.g., comprising about 59.5% of silicon dioxide, 27.5% magnesium oxide, 0.8% dilithium oxide and 2.8% disodium oxide.
   1.3. Any of the foregoing compositions wherein the magnesium alkali metal silicate clay, when in dry form, comprises platelets, e.g., having an average thickness of 0.8 - 1.2 nm, e.g., about 1 nm, and e.g. having an average diameter of 10-30 nm, e.g. about 20 nm.
   1.4. Any of the foregoing compositions wherein the magnesium alkali metal silicate clay is substantially free of aluminum, e.g., comprises less than 5%, e.g., less than 2% aluminum, by dry weight.
   1.5. Any of the foregoing compositions wherein, following incorporation into the dentifrice, the clay remains substantially unhydrated.
   1.6. Any of the foregoing compositions wherein the clay is about 0.2-5%, e.g., about 1 to 4%, or about 2.5% of the composition.
   1.7. Any of the foregoing compositions wherein the clay is Laponite.
   1.8. Any of the foregoing compositions wherein the water insoluble whitening complex is about 0.01 to 5%, more typically about 0.015 to 5%, more particularly about 0.015 to 3% of the composition of the invention.
   1.9. Any of the foregoing compositions wherein the blue dye comprises about 0.1 to 20 %, more particularly about 1 to 10%, and more particularly about 2 to 8 % of the water insoluble whitening complex, and the magnesium alkali metal silicate clay comprises about 80 to 99.9%, more particularly about 90 to 99%, and more particularly about 92 to 98% of the water insoluble whitening complex.
   1.10. Any of the foregoing wherein the triarylmethane dye has a hue angle in the CIELAB system ranging from 180 to 270 degrees.
   1.11. Any of the foregoing compositions wherein the blue dye is a triarylmethane dye, e.g. FD&C Blue #1.
   1.12. Any of the foregoing compositions wherein the orally acceptable carrier comprises a synthetic anionic polymeric polycarboxylate, e.g., in an amount of about 1-5%, e.g., about 2% of the weight of the composition.
   1.13. Any of the foregoing compositions wherein the orally acceptable carrier comprises a 1:4 to 4:1 copolymers of maleic anhydride or acid with another polymerizable ethylenically unsaturated monomer, e.g., in an amount of about 1-5%, e.g., about 2% of the weight of the composition.
   1.14. Any of the foregoing compositions wherein the orally acceptable carrier comprises a methyl vinyl ether/maleic anhydride copolymer having an average molecular weight (M.W.) of about 30,000 to about 1,000,000_{,} e.g. about 300,000 to about 800,000, e.g., in an amount of about 1-5%, e.g., about 2% of the weight of the composition.
   1.15. Any of the foregoing compositions further comprising an effective amount of fluoride, e.g., wherein the fluoride is a salt selected from stannous fluoride, sodium fluoride, potassium fluoride, sodium monofluorophosphate, sodium fluorosilicate, ammonium fluorosilicate, amine fluoride (e.g., N'-octadecyltrimethylendiamine-N,N,N'-tris(2-ethanol)-dihydrofluoride), ammonium fluoride, titanium fluoride, hexafluorosulfate, and combinations thereof.
   1.16. Any of the foregoing compositions comprising L-arginine in free or orally acceptable salt form.
   1.17. Any of the foregoing compositions comprising buffering agents, e.g., sodium phosphate buffer (e.g., sodium phosphate monobasic and disodium phosphate)
   1.18. Any of the foregoing compositions comprising a humectant, e.g., selected from glycerin, sorbitol, propylene glycol, polyethylene glycol, xylitol, and mixtures thereof.
   1.19. Any of the preceding compositions further comprising an abrasive or particulate;
   1.20. The immediately preceding composition wherein the adhesive or particulate is selected from sodium bicarbonate, calcium phosphate (e.g., dicalcium phosphate dihydrate), calcium sulfate, precipitated calcium carbonate, silica (e.g., hydrated silica), iron oxide, aluminum oxide, perlite, plastic particles, e.g., polyethylene, and combinations thereof.
   1.21. Any of the preceding compositions comprising an abrasive in an amount of about 15 wt. % to about 70 wt. % of the total composition weight.
   1.22. Any of the preceding compositions comprising one or more surfactants, e.g., selected from anionic, cationic, zwitterionic, and nonionic surfactants, and mixtures thereof, e.g., comprising an anionic surfactant, e.g., a surfactant selected from sodium lauryl sulfate, sodium ether lauryl sulfate, and mixtures thereof, e.g. in an amount of from about 0.3% to about 4.5% by weight.
   1.23. Any of the preceding compositions further comprising a viscosity modifying amount of one or more polymers selected from polyethylene glycols, polysaccharides (e.g., cellulose derivatives, for example carboxymethyl cellulose, polysaccharide gums, for example xanthan gum or carrageenan gum), and combinations thereof.
   1.24. Any of the preceding compositions in the form of a dentifrice, mouthwash, chewing gum or lozenge.
   1.25. Any of the preceding compositions further comprising flavouring, fragrance and/or coloring.
   1.26. Any of the preceding compositions further comprising water.
   1.27. Any of the foregoing compositions comprising one or more antibacterial agents, for example comprising an antibacterial agent selected from halogenated diphenyl ether (e.g. triclosan), herbal extracts and essential oils (e.g., rosemary extract, tea extract, magnolia extract, thymol, menthol, eucalyptol, geraniol, carvacrol, citral, hinokitol, catechol, methyl salicylate, epigallocatechin gallate, epigallocatechin, gallic acid, miswak extract, sea-buckthorn extract), bisguanide antiseptics (e.g., chlorhexidine, alexidine or octenidine), quaternary ammonium compounds (e.g., cetylpyridinium chloride (CPC), benzalkonium chloride, tetradecylpyridinium chloride (TPC), N-tetradecyl-4-ethylpyridinium chloride (TDEPC)), phenolic antiseptics, hexetidine, octenidine, sanguinarine, povidone iodine, delmopinol, salifluor, metal ions (e.g., zinc salts, for example, zinc citrate, stannous salts, copper salts, iron salts), sanguinarine, propolis and oxygenating agents (e.g., hydrogen peroxide, buffered sodium peroxyborate or peroxycarbonate), phthalic acid and its salts, monoperthalic acid and its salts and esters, ascorbyl stearate, oleoyl sarcosine, alkyl sulfate, dioctyl sulfosuccinate, salicylanilide, domiphen bromide, delmopinol, octapinol and other piperidino derivatives, nicin preparations, chlorite salts; and mixtures of any of the foregoing; e.g., comprising triclosan or cetylpyridinium chloride.
   1.28. Any of the preceding compositions further comprising an additional whitening agent, e.g., a selected from the group consisting of peroxides, metal chlorites, perborates, percarbonates, peroxyacids, hypochlorites, and combinations thereof.
   1.29. Any of the preceding compositions further comprising hydrogen peroxide or a hydrogen peroxide source, e.g., urea peroxide or a peroxide salt or complex (e.g., such as peroxyphosphate, peroxycarbonate, perborate, peroxysilicate, or persulphate salts; for example calcium peroxyphosphate, sodium perborate, sodium carbonate peroxide, sodium peroxyphosphate, and potassium persulfate);
   1.30. Any of the preceding compositions further comprising an agent that interferes with or prevents bacterial attachment, e.g., solbrol or chitosan.
   1.31. Any of the preceding compositions further comprising a source of calcium and phosphate selected from (i) calcium-glass complexes, e.g., calcium sodium phosphosilicates, and (ii) calcium-protein complexes, e.g., casein phosphopeptide-amorphous calcium phosphate
   1.32. Any of the preceding compositions further comprising a soluble calcium salt, e.g., selected from calcium sulfate, calcium chloride, calcium nitrate, calcium acetate, calcium lactate, and combinations thereof.
   1.33. Any of the preceding compositions further comprising a physiologically or orally acceptable potassium salt, e.g., potassium nitrate or potassium chloride, in an amount effective to reduce dentinal sensitivity.
   1.34. Any of the preceding compositions further comprising a breath freshener, fragrance or flavoring.
   1.35. Any of the preceding compositions wherein the composition is a dentifrice and the orally acceptable carrier is a dentifrice base, e.g., comprising one or more carrier polymers, active agents, fluoride ion source, abrasives, foaming agents, surfactants, and/or water, e.g., as hereinbefore set forth.
   1.36. Any of the preceding compositions effective upon application to the oral cavity, e.g., with brushing, to reduce hypersensitivity of the teeth.
   1.37. A composition obtained or obtainable by combining the ingredients as set forth in any of the preceding compositions.
   1,38. Any of the preceding compositions in the form of a toothpaste.
   1.39. Any of the preceding compositions further comprising effective amounts of additional agents selected from fluoride, 1-arginine in free or orally acceptable salt form, antibacterial agents, anti-inflammatory compounds, and whitening agents.
   1.40. Any of the preceding compositions wherein the composition is a toothpaste or mouthwash optionally further comprising one or more of one or more of water, abrasives, surfactants, foaming agents, vitamins, polymers, enzymes, humectants, thickeners, antimicrobial agents, preservatives, flavorings, colorings and/or combinations thereof.
   1.41. Any of the preceding compositions wherein the composition is toothpaste.

The invention further provides the use of a water insoluble whitening complex comprising (a) a triarylmethane dye and (b) a magnesium alkali metal silicate clay, in the manufacture of an oral care composition, e.g., any of Composition 1, *et seq*., e.g., for use in any of the methods as described in the preceding paragraph.

The invention further provides methods of manufacturing a dentifrice composition, e.g., Composition 1, et seq., comprising (i) forming a water insoluble whitening complex by combining (a) a triarylmethane dye and (b) a magnesium alkali metal silicate clay, and combining the water insoluble whitening complex thus formed with a dentifrice base.

### EXAMPLES

### Example 1

Two (2) grams of Laponite XLG is dispersed into 73 grams of DI water with agitation. 0.05 gram of FD&C Blue # 1 is dissolved into 25 gram of water in another beaker to form dye solution. When the Laponite mixture turns translucent, the dye solution is added with agitation until a uniform translucent mixture is formed. Two (2) grams of NaCl is then added with agitation until it is fully dissolved. The resulting mixture is then transferred to centrifuge tubes and centrifuged at 9000 rpm for 15min. The dye/Laponite water insoluble complex is formed and separated from the aqueous media.

### Example 2

### Procedure for dye absorption:

1. Disperse 1.0 gram of a clay sample into 74.0 gram of water. Keep stirring to get uniform dispersion
2. In another beaker, dissolve 0.05g FD&C Blue#1 and 2.0g of NaCl into 23.0 gram of water.
3. Add the dye solution into the clay dispersion with continuous agitation.
4. Mix for 5 min after adding dye solution
5. Centrifuge the mixture at 7000rpm for 5min.
6. Take the supernatant for UV analysis to calculate blue absorption %.

### Procedure for dye desorption (for SBH REM, BL865, and Laponite only):

1. Add 15.0g of DI water into the separated clay/dye complex
2. Vortex for 2 minutes or until the complex fully dispersed
3. Centrifuge for 5 min at 7000 rpm.
4. Record visual observation on dye desorption

The results are in the following table:

| | Supplier | % of dye being absorbed | desorption |
|---|---|---|---|
| Argel Kaolin Mineral 90W (kaolinite) | Arclay | 0 | |
| Argel SBH R.E.M. (bentonite) | Arclay | 99 | most |
| Argel CBH White R.E.M. (kaolinite) | Arclay | 14 | |
| RXG 7254 (bentonite) | Southern Clay | 89 | |
| BL 865 (bentonite) | Southern Clay | 98 | most |
| GWH (bentonite) | Southern Clay | 76 | |
| Laponite | Southern Clay | 99.97 | little |

The laponite clay is the only clay tested which both absorbs and retains the dye.

As those skilled in the art will appreciate, numerous changes and modifications may be made to the embodiments described herein without departing trorn the spirit of the invention,

## Claims

1. A tooth whitening oral care composition comprising:
(i) a whitening complex, wherein the complex comprises
(a) a magnesium alkali metal silicate clay, wherein the clay is a hectorite clay; and
(b) triarylmethane dye as an orally acceptable blue dye,
(ii) an orally acceptable carrier; and
(iii) L-arginine in free or orally acceptable salt form;
wherein the complex is prepared by:
dispersing the clay into an aqueous solution, combining the aqueous solution of clay with an aqueous solution of the dye, adding salt, optionally sodium chloride, under agitation, and isolating the complex from the liquid phase.

2. The composition of claim 1 wherein the hectorite clay is a synthetic clay comprising 58-61% silicon dioxide, 26-29% magnesium oxide, 0.7-0.9% dilithium oxide and 2.6-3% disodium oxide.

3. The composition of any of the foregoing wherein the magnesium alkali metal silicate clay, when in dry form, comprises platelets having an average thickness of 0.8 - 1.2 nm.

4. The composition of any of the foregoing claims wherein the triarylmethane dye is a dye having a blue to blue-green color with a hue angle in the CIELAB system selected from the ranges consisting of from 180 to 270 degrees and from 180 to 200 degrees.

5. The composition of any of the foregoing wherein the magnesium alkali metal silicate clay is a synthetic hectorite clay comprising 59.5% of silicon dioxide, 27.5% magnesium oxide, 0.8% dilithium oxide and 2.8% disodium oxide.

6. The composition of any of the foregoing claims wherein the whitening complex is about 0.015 - 3% of the composition.

7. The composition of any of the foregoing claims wherein the orally acceptable carrier comprises a synthetic anionic polymeric polycarboxylate.

8. The composition of claim 7 wherein the synthetic anionic polymeric polycarboxylate is a methyl vinyl ether/maleic anhydride having an average molecular weight of about 30,000 to about 1,000,000.

9. The composition of claim 7 or 8 wherein the synthetic anionic polymeric polycarboxylate is about 1-5% of the weight of the composition.

10. The composition according to any of the foregoing claims further comprising an effective amount of an additional agent selected from fluoride, an additional antibacterial agent, an anti-inflammatory agent, a whitening agent, and a combination of two or more thereof.

11. The composition of any of the foregoing claims in the form of a dentifrice.

12. The composition according to any of the foregoing claims wherein the composition is a toothpaste comprising one or more of water, an abrasive, a surfactant, a foaming agent, a vitamin, a polymer, an enzyme, a humectant, a thickener, an antimicrobial agent, a preservative, a flavoring, a coloring and/or a combination of two or more thereof.

13. The composition according to claim 1 for use in a method for whitening teeth and/or for treating or reducing dental hypersensitivity comprising applying to the oral cavity an effective amount of said composition.

14. Use of a whitening complex comprising (a) a triarylmethane dye and (b) a magnesium alkali metal silicate clay in the manufacture of an oral care composition; wherein the clay is a hectorite clay, and wherein the oral care composition further comprises L-arginine in free or orally acceptable salt from; and wherein the complex is prepared by: dispersing the clay into an aqueous solution, combining the aqueous solution of clay with an aqueous solution of the dye, adding a salt, optionally sodium chloride, under agitation, and isolating the complex from the liquid phase.

## Patentansprüche

1. Zahnaufhellungsmundpflegezusammensetzung umfassend:
(i) einen Aufhellungskomplex, worin der Komplex umfasst:
(a) einen Magnesiumalkalimetallsilikatlehm, worin der Lehm ein Hektoritlehm ist und
(b) Triarylmethanfarbstoff als oral annehmbaren blauem Farbstoff,
(ii) einem oral annehmbaren Träger und
(iii) L-Arginin in freier oder oral annehmbarer Salzform;
worin der Komplex hergestellt ist durch:
Dispergieren des Lehms in einer wässrigen Lösung, Kombinieren der wässrigen Lösung des Lehms mit einer wässrigen Lösung des Farbstoffes, Zugabe von Salz ggf. Natriumchlorid, unter Rühren und Isolieren des Komplexes aus der flüssigen Phase.

2. Zusammensetzung gemäß Anspruch 1, worin der Hektoritlehm ein synthetischer Lehm ist, umfassend 58-61% Siliziumdioxid, 26-29% Magnesiumoxid, 0,7-0,9% Dilithiumoxid und 2,6-3% Dinatriumoxid.

3. Zusammensetzung nach irgendeinem der vorhergehenden Ansprüche, worin der Magnesiumalkalisilikatlehm, wenn in trockener Form vorliegend, Plättchen mit einer durchschnittlichen Dicke von 0,3 - 1,2 nm umfasst.

4. Zusammensetzung nach irgendeinem der vorhergehenden Ansprüche, worin der Triarylmethanfarbstoff ein Farbstoff mit einer blauen bis blau-grünen Farbe ist, mit einem Farbton im CIELAB-System ausgewählt aus den Bereichen bestehend aus von 180-270 Grad und von 180-200 Grad.

5. Zusammensetzung nach irgendeinem der vorhergehenden Ansprüche, worin der Magnesiumalkalimetallsilikatlehm ein synthetischer Hektoritlehm ist, umfassend 59,5% Siliziumdioxid, 27,5% Magnesiumoxid, 0,8% Dilithiumoxid und 2,8% Dinatriumoxid.

6. Zusammensetzung nach irgendeinem der vorhergehenden Ansprüche, worin der Aufhellungskomplex etwa 0,01-3% der Zusammensetzung ist.

7. Zusammensetzung nach irgendeinem der vorhergehenden Ansprüche, worin der oral annehmbare Träger ein synthetisches, anionisches, polymeres Polycarboxylat umfasst.

8. Zusammensetzung nach irgendeinem der vorhergehenden Ansprüche, worin das synthetische, anionische, polymere Polycarboxylat ein Methylvinylether/Maleinsäureanhydrid ist mit einem durchschnittlichen Molekulargewicht von etwa 30.000 bis etwa 1.000.000.

9. Zusammensetzung nach Anspruch 7 oder 8, worin das synthetische, anionische, polymere Polycarboxylat etwa 1-5% des Gewichts der Zusammensetzung ist.

10. Zusammensetzung nach irgendeinem der vorhergehenden Ansprüche, weiterhin umfassend, eine wirksame Menge eines zusätzlichen Mittels ausgewählt aus Fluorid, einem zusätzlichen antibakteriellen Mittel, einem Antiinflammatorischen Mittel, einem Aufhellungsmittel und einer Kombination aus zwei oder mehreren davon.

11. Zusammensetzung nach irgendeinem der vorhergehenden Ansprüche, in Form eines Zahnputzmittels.

12. Zusammensetzung nach irgendeinem der vorhergehenden Ansprüche, worin die Zusammensetzung eine Zahnpasta ist, umfassend ein oder mehrere aus Wasser, einem Abrasivstoff, einem Tensid, einem Schäumungsmittel, einem Vitamin, einem Polymer, einem Enzym, einem Befeuchtungsmittel, einem Verdickungsmittel, einem antimikrobiriellen Mittel, einem Konservierungsstoff, einem Geschmacksmittel, einem Farbmittel und/oder einer Kombination aus zwei oder mehreren davon.

13. Zusammensetzung nach Anspruch 1 zur Verwendung in einem Verfahren zur Verwendung zur Aufhellung von Zähnen und/oder zur Behandlung oder Reduzierung von Dentalüberempfindlichkeit und umfassend die Verwendung einer wirksamen Mengen der Zusammensetzung in der Mundhöhle.

14. Verwendung eines Aufhellungskomplexes, umfassend (a) ein Triarylmethan Farbstoff und (b) ein Magnesiumalkalimetallsilikatlehm in der Herstellung einer Mundpflegezusammensetzung; wobei der Lehm ein Hektoritlehm ist, und wobei die Mundpflegezusammensetzung weiterhin umfasst L-Arginin in freier oder oral annehmbarer Salzform; und wobei der Komplex hergestellt ist durch:
Dispergieren des Lehms in eine wässrigen Lösung, Kombinieren der wässrigen Lösung des Lehms mit einer wässrigen Lösung des Farbstoffs, Zugabe eines Salzes, ggf. Natriumchlorid, unter Rühren und Isolieren des Komplexes aus der flüssigen Phase.

## Revendications

1. Composition de soin buccal de blanchiment des dents comprenant :
(i) un complexe de blanchiment, dans lequel le complexe comprend
(a) une argile de silicate de métal alcalin et de magnésium, dans laquelle l'argile est une argile hectorite ; et
(b) un colorant triarylméthane en tant que colorant bleu acceptable par voie orale,
(ii) un support acceptable par voie orale ; et
(iii) de la L-arginine sous forme libre ou sous forme de sel acceptable par voie orale ;
dans laquelle le complexe est préparé par :
la dispersion de l'argile dans une solution aqueuse, la combinaison de la solution aqueuse d'argile avec une solution aqueuse du colorant, l'ajout de sel, éventuellement de chlorure de sodium, sous agitation, et l'isolement du complexe de la phase liquide.

2. Composition selon la revendication 1, dans laquelle l'argile hectorite est une argile synthétique comprenant 58 à 61 % de dioxyde de silicium, 26 à 29 % d'oxyde de magnésium, 0,7 à 0,9 % d'oxyde de dilithium et 2,6 à 3 % d'oxyde disodique.

3. Composition selon l'une quelconque des revendications précédentes, dans laquelle l'argile de silicate de métal alcalin et de magnésium, lorsqu'elle est sous forme sèche, comprend des plaquettes ayant une épaisseur moyenne de 0,8 à 1,2 nm.

4. Composition selon l'une quelconque des revendications précédentes, dans laquelle le colorant triarylméthane est un colorant ayant une couleur bleue à bleu-vert avec un angle de teinte dans le système CI ELAB choisi parmi les plages constituées de 180 à 270 degrés et de 180 à 200 degrés.

5. Composition selon l'une quelconque des revendications précédentes, dans laquelle l'argile de silicate de métal alcalin et de magnésium est une argile hectorite synthétique comprenant 59,5 % de dioxyde de silicium, 27,5 % d'oxyde de magnésium, 0,8 % d'oxyde de dilithium et 2,8 % d'oxyde disodique.

6. Composition selon l'une quelconque des revendications précédentes, dans laquelle le complexe de blanchiment représente environ 0,015 à 3 % de la composition.

7. Composition selon l'une quelconque des revendications précédentes, dans laquelle le support acceptable par voie orale comprend un polycarboxylate polymère anionique synthétique.

8. Composition selon la revendication 7, dans laquelle le polycarboxylate polymère anionique synthétique est un éther méthylvinylique/anhydride maléique ayant un poids moléculaire moyen d'environ 30 000 à environ 1 000 000.

9. Composition selon la revendication 7 ou 8, dans laquelle le polycarboxylate polymère anionique synthétique représente environ 1 à 5 % du poids de la composition.

10. Composition selon l'une quelconque des revendications précédentes, comprenant en outre une quantité efficace d'un agent supplémentaire choisi parmi le fluorure, un agent antibactérien supplémentaire, un agent anti-inflammatoire, un agent de blanchiment et une combinaison de deux ou plus de ceux-ci.

11. Composition selon l'une quelconque des revendications précédentes sous la forme d'un dentifrice.

12. Composition selon l'une quelconque des revendications précédentes, dans laquelle la composition est une pâte dentifrice comprenant un ou plusieurs éléments parmi l'eau, un abrasif, un tensioactif, un agent moussant, une vitamine, un polymère, une enzyme, un humectant, un épaississant, un agent antimicrobien, un conservateur, un arôme, un colorant et/ou une combinaison de deux ou plus de ceux-ci.

13. Composition selon la revendication 1 pour une utilisation dans un procédé de blanchiment des dents et/ou pour le traitement ou la réduction de l'hypersensibilité dentaire comprenant l'application à la cavité buccale d'une quantité efficace de ladite composition.

14. Utilisation d'un complexe de blanchiment comprenant (a) un colorant triarylméthane et (b) une argile de silicate de métal alcalin et de magnésium dans la fabrication d'une composition de soin buccal ; dans laquelle l'argile est une argile hectorite, et dans laquelle la composition de soin buccal comprend en outre de la L-arginine sous forme libre ou sous forme de sel acceptable par voie orale ; et dans laquelle le complexe est préparé par : la dispersion de l'argile dans une solution aqueuse, la combinaison de la solution aqueuse d'argile avec une solution aqueuse du colorant, l'ajout de sel, éventuellement de chlorure de sodium, sous agitation, et l'isolement du complexe de la phase liquide.
